# FASCICULE DE BREVET EUROPEEN

(11) **EP 0 900 559 B1**
(45) Date de publication et mention de la délivrance du brevet: **03.01.2001**
(21) Numéro de dépôt: 98401640.2
(22) Date de dépôt: 01.07.1998
(51) Int. Cl.: A61K 7/00, A61K 9/107

(54) **Utilisation d'une dispersion à base de vésicules lipidiques comme composition anti-inflammatoire**
Verwendung einer Dispersion, die auf Lipidvesikeln basiert ist, als entzündungshemmende Zusammensetzung
Use of a dispersion based on lipid vesicles as an anti-inflammatory composition

(30) Priorité: 27.08.1997 FR 9710709
(43) Date de publication de la demande: 10.03.1999
(73) Titulaire: L'OREAL, 75008 Paris (FR)
(72) Inventeur: Simonnet, Jean-Thierry, 75011 Paris (FR)
(74) Mandataire: Tezier Herman, Béatrice

(56) Documents cités:
- EP-A- 0 641 557
- EP-A- 0 705 593
- EP-A- 0 832 643
- WO-A-96/16545
- FR-A- 2 679 788

## Description

La présente invention concerne l'utilisation d'une dispersion aqueuse de globules huileux pourvus chacun d'un enrobage cristal liquide monolamellaire ou oligolamellaire, dans une composition cosmétique ou pour la préparation d'une composition dermatologique, pour prévenir et/ou combattre les désordres cutanés faisant intervenir un processus inflammatoire, notamment pour prévenir et/ou traiter une irritation cutanée et/ou pour prévenir et/ou traiter un érythème solaire.

L'invention se rapporte aussi à un procédé de traitement cosmétique d'une inflammation, d'une irritation et/ou d'un érythème solaire, par application d'une telle dispersion.

L'inflammation (ou processus inflammatoire) est un ensemble de réactions biologiques qu'on retrouve dans toute l'échelle animale. Chez l'homme, deux malades sur trois présentent un syndrome inflammatoire. L'inflammation peut être localisée. Elle peut se définir comme la première réponse à toute agression locale par une série de réactions non spécifiques déclenchées quelle que soit la cause initiale et se déroulant en trois temps : vasculaire, cellulo-vasculaire et fibrose tissulaire.

L'inflammation localisée et notamment cutanée se caractérise par des symptômes tels que le gonflement, la douleur, la rougeur et la chaleur.

A titre de phénomènes inflammatoires cutanés, on peut citer par exemple les érythèmes, en particulier dus aux ultraviolets et au soleil, le prurit, l'érythème noueux, l'urticaire, la mastocytose systémique, le psoriasis, les piqûres d'insectes, d'autres affections dermatologiques comme la polychondrite atrophiante, l'érythèmalgie, la nécrobiose lipoïdique, le lupus érythémateux disséminé ou encore toute irritation cutanée due au contact avec des produits agressifs tels que certains produits ménagers.

On recherche depuis de nombreuses années, dans l'industrie pharmaceutique et cosmétique, des substances permettant de prévenir ou de traiter l'inflammation. A cet égard, nombreuses sont celles qui ont déjà été décrites dans la littérature sous les appellations d'anti-inflammatoires stéroïdiens ou non-stéroïdiens (AIS ou AINS) et dont on trouvera une description dans, par exemple, l'ouvrage de Schorderet et Dayer "Pharmacologie, Des concepts fondamentaux aux applications thérapeutiques", 1992, chapitre 37, pages 541-561, 2^{ième} édition, Frison-Roche/Slatkine éditeurs.

Outre que les anti-inflammatoires connus présentent souvent des effets secondaires non négligeables, il demeure intéressant de disposer de nouveaux produits à activité anti-inflammatoire.

Le document WO-A-96/16545 décrit une composition de soin sous forme d'émulsion huile-dans-eau contenant un émulsifiant amphiphile formant des cristaux liquides, et le document EP-A-832643 décrit la stabilisation de rétinoïde dans des émulsion huile-dans-eau contenant une couche de barrière cristalline entre les gouttes d'huile et la phase aqueuse.

Le but de la présente invention est donc de pouvoir disposer d'un produit nouveau présentant une activité anti-inflammatoire tout en ne présentant pas d'effets secondaires notables.

La Demanderesse a trouvé de façon surprenante qu'une dispersion à base de globules huileux enrobés d'une phase cristal liquide lamellaire, et plus particulièrement monolamellaire ou oligolamellaire, avait des propriétés anti-inflammatoires et permettait de prévenir une réaction inflammatoire. La dispersion selon l'invention peut en particulier être utilisée pour prévenir et/ou traiter l'érythème solaire. En outre, la dispersion selon l'invention peut être utilisée quand la réaction inflammatoire est provoquée par une irritation cutanée induite par contact avec un produit irritant.

Cette découverte est à la base de l'invention.

La présente invention a donc pour objet l'utilisation d'une dispersion aqueuse de globules huileux pourvus chacun d'un enrobage cristal liquide monolamellaire ou oligolamellaire, comme actif ayant des propriétés anti-inflammatoires, pour la préparation d'une composition dermatologique destinée à prévenir et/ou traiter une irritation cutanée et/ou à traiter les peaux sensibles et/ou à prévenir et/ou combattre les désordres cutanés faisant intervenir un processus inflammatoire et/ou à prévenir et/ou traiter un érythème solaire, le dit enrobage cristal liquide étant obtenu :
1) soit à partir d'au moins un agent tensioactif lipophile de HLB 2 à 5, d'au moins un agent tensioactif hydrophile de HLB 8 à 12 et d'au moins un acide gras, la dispersion contenant un agent basique à l'état dissous dans une phase aqueuse ;
2) soit à partir d'au moins un agent tensioactif lipophile de HLB 2 à 5, d'au moins un agent tensioactif hydrophile de HLB 8 à 12 et d'au moins un lipide amphiphile ionique conférant à la composition un pH allant de 5,5 à 7,5.

La composition cosmétique ou dermatologique contenant la dispersion utilisable selon l'invention comporte un milieu physiologiquement acceptable, c'est-à-dire compatible avec la peau, le cuir chevelu et les cheveux.

Les globules huileux de l'invention comportent une membrane mono- ou oligolamellaire de lipides ioniques ou non ioniques renfermant une phase huileuse (vésicules lipidiques à coeur huileux). On entend par couche oligolamellaire une couche comprenant de 2 à 5 lamelles lipidiques. La taille moyenne des globules huileux revêtus est inférieure à 500 nanomètres et, par exemple, de l'ordre de 200 nanomètres. Du fait de leur petite taille, la pénétration des globules dans lés espaces intercornéocytaires qui sont de taille comparable est grandement facilitée.

La dispersion selon l'invention est du type émulsion, notamment huile dans eau (H/E), contenant les globules huileux pourvus d'un enrobage cristal liquide lamellaire. En particulier, ces émulsions sont du type de celles décrites dans les demandes de brevet européen EP-A-0 641 557 et EP-A-0 705593.

Ainsi, et selon un premier mode préféré ue réalisation de l'invention, les globules huileux sont du type de ceux décrits dans la demande de brevet EP-A-0 641 557 déposée au nom de la Demanderesse. Ainsi, conformément à cette demande, chaque globule huileux est unitairement enrobé d'une couche monolamellaire ou oligolamellaire obtenue à partir d'au moins un agent tensioactif lipophile, d'au moins un agent tensioactif hydrophile et d'au moins un acide gras, et la phase aqueuse de l'émulsion selon l'invention contient à l'état dissous un agent basique.

L'agent tensioactif lipophile peut être choisi dans le groupe comprenant le distéarate de saccharose, le distéarate de diglycéryle, le tristéarate de tétraglycéryle, le décastéarate de décaglycéryle, le monostéarate de diglycérol, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthylèné 2 OE, (comprenant 2 motifs d'oxyde d'éthylène), le mono et dibéhénate de glycéryle, le tétrastéarate de pentaérythritol. De préférence, l'agent tensioactif lipophile est le distéarate de saccharose.

L'agent tensioactif lipophile présente de préférence un HLB (balance hydrophilelipophile) allant de 2 à 5.

L'agent tensioactif hydrophile peut être choisi dans le groupe comprenant le monostéarate de sorbitane polyoxyéthyléné à 4 moles d'oxyde d'éthylène (Polysorbate 61), le tristéarate de sorbitane polyoxyéthyléné à 20 moles d'oxyde d'éthylène, le monostéarate polyoxyéthyléné à 8 moles d'oxyde d'éthylène, le monostéarate d'hexaglycéryle, le monostéarate polyoxyéthyléné à 10 moles d'oxyde d'éthylène, le distéarate polyoxyéthyléné à 12 moles d'oxyde d'éthylène, le distéarate de méthylglucose polyoxyéthyléné à 20 moles d'oxyde d'éthylène. De préférence, l'agent tensioactif hydrophile est le Polysorbate 61.

L'agent tensioactif hydrophile présente de préférence un HLB allant de 8 à 12.

Selon une variante de réalisation de l'invention, l'agent tensioactif lipophile et l'agent tensioactif hydrophile comportent chacun au moins une chaîne grasse saturée ayant plus de 12 atomes de carbone, de préférence allant de 16 à 22 atomes de carbone.

Selon une variante de réalisation de l'invention, l'acide gras comporte au moins une chaîne grasse saturée ayant plus de 12 atomes de carbone, de préférence allant de 16 à 22 atomes de carbone. De préférence, l'acide gras est choisi dans le groupe comprenant l'acide palmitique, l'acide stéarique, l'acide arachidique et l'acide béhénique. Encore plus préférentiellement, l'acide gras est l'acide stéarique.

L'agent basique peut être choisi parmi les bases minérales et les bases organiques, notamment les bases amphotères, c'est-à-dire des bases ayant à la fois des groupements fonctionnels anioniques et cationiques. Des exemples d'agents basiques peuvent être la soude, la triéthanolamine, la lysine ou encore l'arginine. De préférence, l'agent basique est la lysine.

L'agent basique est dissous dans la phase aqueuse en une quantité au moins égale à la quantité nécessaire à la neutralisation de l'acide gras.

Selon un second mode préféré de réalisation de l'invention, les globules huileux sont du type de ceux décrits dans la demande de brevet EP-A-0 705593 déposée au nom de la Demanderesse. Ainsi, conformément à cette demande, les globules huileux sont unitairement enrobés d'une couche monolamellaire ou oligolamellaire obtenue à partir d'au moins un agent tensioactif lipophile et d'au moins un agent tensioactif hydrophile tels que ceux définis ci-dessus, et également à partir d'au moins un lipide amphiphile ionique conférant à la composition un pH allant de 5,5 à 7,5.

Le lipide amphiphile ionique mis en oeuvre dans le cadre de la présente invention est de préférence choisi dans le groupe comprenant les lipides anioniques neutralisés, les lipides amphotères et les dérivés alkylsulfoniques.

Les lipides anioniques neutralisés sont choisis en particulier parmi :
- les sels alcalins du dicétylphosphate, et en particulier les sels de sodium et de potassium ;
- les sels alcalins du dimyristylphosphate, et en particulier les sels de sodium et de potassium ;
- les sels alcalins du cholestérol sulfate, et en particulier le sel de sodium ;
- les sels alcalins du cholestérol phosphate, et en particulier le sel de sodium ;
- les sels monosodique et disodique des acides acylglutamiques, et en particulier les sels monosodique et disodique de l'acide N-stéaroyl glutamique ;
- le sel de sodium de l'acide phosphatidique.

Les lipides amphotères sont choisis en particulier parmi les phospholipides et notamment la phosphatidyléthanolamine de soja pure.

Les dérivés alkylsulfoniques sont avantageusement les composés de formule : 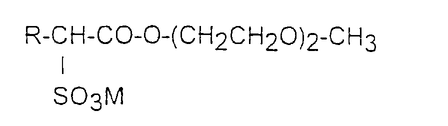 dans laquelle R représente les radicaux C₁₆H₃₃ et C₁₈H₃₇ pris en mélange ou séparément, et M est un métal alcalin, de préférence le sodium.

De préférence, le lipide amphiphile ionique est le sel monosodique de l'acide N-stéaroyl glutamique vendu sous la dénomination commerciale « Amisoft HS 11 » par Ajinomoto ou bien le sel disodique de l'acide N-stéaroyl glutamique vendu sous la dénomination commerciale « Amisoft HS 21 » par Ajinomoto.

L'enrobage selon le second mode de réalisation de l'invention des globules huileux est obtenu de préférence par l'utilisation d'une quantité totale d'agent tensioactif hydrophile, d'agent tensioactif lipophile et de lipide amphiphile ionique, comprise entre environ 2% et environ 6% en poids par rapport au poids total de la composition. Encore plus préférentiellement, cette quantité est comprise entre 3% et 4%. Les quantités relatives de tensioactifs lipophile, hydrophile et de lipide amphiphile ionique varient de préférence dans les fourchettes respectives suivantes : 35-55% / 25-40% / 15-35% en poids par rapport à leur poids total.

La phase grasse, et en particulier celle des globules huileux enrobés, représente de préférence 5 à 50 % en poids par rapport au poids total de la composition. Encore plus préférentiellement, ce pourcentage est compris entre 10 et 40 %. De préférence, le rapport huile/eau est égal ou inférieur à 1.

Le rapport pondéral des globules huileux aux éléments constitutifs de l'enrobage est de préférence de 3 à 13, plus préférentiellement de 5 à 8 ; encore plus préférentiellement ce rapport est égal à 7 environ.

Les huiles pouvant être utilisées dans la phase grasse sont les huiles utilisées classiquement dans les compositions cosmétiques ou dermatologiques comme par exemple les huiles animales, végétales (huile de tournesol), minérales ou de synthèse, les huiles de silicone (cyclométhicone), les huiles fluorées et perfluorées. La phase grasse peut comprendre en outre des alcools gras, des esters d'acides gras et des cires (cire hydrogénée de lait).

Les dispersions utilisées dans l'invention peuvent contenir en outre dans les globules huileux une substance grasse ou lipophile choisie parmi les agents antioxydants, antiradicaux libres, mélanorégulateurs, accélérateurs de bronzage, dépigmentants, de coloration de la peau, liporégulateurs, amincissants, anti-acnéiques, antiséborrhéiques, antivieillissement, antirides, anti-UV, kératolytiques, anti-inflammatoires, rafraîchissants, cicatrisants, protecteurs vasculaires, antibactériens, antifongiques, antitranspirants, déodorants, conditionneurs des cheveux, immunomodulateurs, nourrissants, les huiles essentielles et les parfums.

On peut citer comme exemples d'actifs lipophiles pour le traitement de la peau et/ou des cheveux, utilisables dans le cadre de la présente invention les composés suivants :

D α tocophérol, DL α tocophérol, acétate de D α tocophérol, acétate de DL α tocophérol, palmitate d'ascorbyle, glycérides de vitamine F, vitamines D et notamment vitamine D₂ et vitamine D₃, rétinol, esters de rétinol (palmitate de rétinol, propionate de rétinol), β carotène, D panthénol, farnésol, acétate de farnésyle, huiles riches en acides gras essentiels et notamment huiles de jojoba et de cassis, acide n octanoyl-5 salicylique, acide salicylique, alkylesters d'α-hydroxyacides tels que acide citrique, acide lactique et acide glycolique, acide asiatique, acide madécassique, asiaticoside, extrait total de Centella Asiatica, acide β glycyrrhétinique, α bisabolol, céramides et notamment le 2 oléoylamino-1,3 octadécane, phytanetriol, sphingomyéline de lait, phospholipides d'origine marine riches en acides gras essentiels polyinsaturés, éthoxyquine, extrait de romarin, extrait de mélisse, quercetine, extrait de microalgues séchées (Algoxan red commercialisé par Algatec), huile essentielle de bergamotte, octylmethoxycinnamate (Parsol MCX commercialisé par Givaudan-Roure), butylméthoxydibenzoylméthane (Parsol 1789 commercialisé par Givaudan-Roure), octyl triazone (Uvinul T150 commercialisé par BASF), oxydes de fer jaune, brun, noir, rouge, oxydes de titane, pouvant se présenter sous forme micrométrique ou nanométrique ou sous forme enrobée (par exemple par un perfluoroalkyl), di-tertiobutyl-3,5 hydroxy-4 benzylidène-3 camphre, 2-benzotriazol -2-yl-4-méthyl-6-[3-[1,3,3,3-tétraméthyl-1-[(triméthylsilyl)oxy]-disiloxanyl]-2-méthyl -propyl]phénol, huile perfluorée (perfluorodécaline, perfluorooctyl bromure), huile de maïs hyperoxygénée (Epaline 100 commercialisée par la société Carilene).

Les compositions utilisées dans l'invention peuvent comprendre en outre des adjuvants classiques dans le domaine cosmétique ou dermatologique, notamment choisis parmi les solvants organiques, les épaississants ioniques ou non ioniques, les adoucissants, les antioxydants, les opacifiants, les stabilisants, les émollients, les agents antimousse, les parfums, les conservateurs, les tensioactifs, les charges, les séquestrants (EDTA), les polymères, les propulseurs, les agents alcalinisants ou acidifiants, les colorants, les actifs hydrophiles ou lipophiles ou tout autre ingrédient habituellement utilisé en cosmétique ou dans le domaine de la dermatologie. Les quantités de ces différents adjuvants sont celles classiquement utilisées dans les domaines considérés, et par exemple de 0,01 à 20 % du poids total de la composition. Bien entendu, ces adjuvants doivent être de nature et utilisés en quantité telle qu'ils ne perturbent pas la dispersion de l'invention.

Parmi les solvants organiques, on peut citer les alcools et polyols inférieurs.

Les épaississants peuvent être choisis notamment parmi les polymères carboxyvinyliques, les gommes de guar et les celluloses modifiées ou non, comme par exemple la gomme de guar hydroxypropylée, la gomme de xanthane, la méthylhydroxyéthylcellulose, l'hydroxypropylméthylcellulose ou encore l'hydroxyéthylcellulose.

Ces compositions peuvent comprendre en particulier un ou plusieurs composés cosmétiquement ou dermatologiquement actifs, hydrophiles ou lipophiles, libres ou encapsulés dans des vésicules, choisis par exemple parmi les hydratants (glycérine, propylène glycol, hydroxyproline), les vitamines, les agents kératolytiques (hydroxyacides).

Les compositions utilisées dans l'invention peuvent être utilisées par exemple comme compositions solaires ou après-soleil sur l'épiderme humain ou sur les cheveux. Elles peuvent se présenter sous la forme de crèmes, de laits, ou encore de lotions fluides.

Des exemples concrets, mais nullement limitatifs, illustrant le procédé et les compositions conformes à l'invention, vont maintenant être donnés.

Des exemples concrets, mais nullement limitatifs, illustrant le procédé et les compositions conformes à l'invention, vont maintenant être donnés.

### Exemple 1 : Composition apaisante

### Phase A :

- Distéarate de saccharose commercialisé par la Société "STEARINERIE DUBOIS" 2 %
- Polysorbate 61 commercialisé sous le nom de "TWEEN 61" par la Société ICI 1,3 %
- Acide stéarique 1 %
- Stearyl heptanoate /stearyl octanoate 5 %
- Cire hydrogénée du lait commercialisé par ICHIMARU PHARCO sous le nom de Milkwhity 3,2 %
- Farnesol / acétate de farnesyl (mélange commercialisé par INDUCHEM sous le nom de Unibiovit B33) 3 %
- Cyclométhicone 3,7 %
- Huile de tournesol 10 %
- Acétate de D a tocophérol 1 %
- Conservateur 0,15 %

### Phase B :

- Glycérine 3 %
- EDTA 0,05 %
- Hydroxyproline 1 %
- Lysine 0,5 %
- Propylène glycol 0,7 %
- Conservateur 0,4 %
- Eau déminéralisée 55 %

### Phase C :

- Gomme de silicone commercialisé par DOW CORNING sous le nom "Q2-1403 Fluid" 4 %

### Phase D :

- Polymère carboxyvinylique commercialisé par la Société SIGMA sous le nom "SYNTHALEN K" 0,1 %
- Eau déminéralisée qsp 100 %

Le procédé de fabrication consiste à porter la phase A et la phase B à 75°C, à introduire rapidement la phase B dans la phase A sous vive agitation et à maintenir la température de 75°C et l'agitation pendant 15 à 30 minutes. On soumet ensuite le mélange par trois fois à une homogénéisation sous haute pression (500.10⁵ Pa) (500 bars). On ramène alors la température du mélange à 25°C et on introduit la phase C sous vive agitation. Ensuite, on soumet de nouveau le mélange à une homogénéisation sous haute pression (300.10⁵ Pa) (300 bars).

On obtient une suspension blanche, laiteuse, très fluide, dans laquelle on introduit la phase D prédispersée.

La crème blanche obtenue, lisse et brillante, est apte à prévenir ou traiter les irritations cutanées provoquées par exemple par les produits ménagers ou les érythèmes solaires.

Le test suivant met en évidence l'efficacité de la dispersion utilisée selon l'invention. Ce test est réalisé *in vivo,* sur un panel de 10 sujets sensibles à une solution de laurylsulfate de sodium (initiateur de l'irritation). Il consiste à mesurer l'évolution de la perte insensible en eau et l'évolution de la microvascularisation au vélocimètre laser doppler.

La perte insensible en eau (PIE ou TEWL en anglais) a été mesurée à l'aide d'un évaporimètre (Servomed) qui est un appareil permettant la détermination quantitative de l'évaporation d'eau à partir d'une surface de peau (par exemple de l'avant-bras) selon la méthode décrite par NILSSON GE "Measurement of water exchange through skin", dans Med. Biol. Eng. Comput. 1977 ; 15 : 208 - 18. Des capteurs permettent de mesurer la pression partielle de la vapeur d'eau en deux points situés à des distances différentes de la surface de la peau. On peut déterminer ainsi le gradient de pression partielle de vapeur d'eau entre les deux points, et donc le taux d'évaporation, conformément à la loi de Fick.

La composition utilisée dans l'invention a été appliquée deux fois par jour pendant 4 jours sur l'avant-bras des sujets. Puis, une solution de laurylsulfate de sodium a été appliquée sous patch occlusif pendant 24 heures. Les mesures de PIE et au vélocimètre laser doppler sont effectuées 3 heures après le retrait du patch. Les résultats sont réunis dans la tableau suivant :

| | PIE | Velocimétrie laser Doppler |
|---|---|---|
| Peau nue | 41,6 | 401 |
| Composition selon l'invention | 34,01 | 226 |
| % dévolution | - 18 % | - 43,96 % |

Ces résultats montrent que l'application de la composition selon l'invention a un effet préventif de l'irritation, très important.

Pour étudier l'effet curatif de la composition utilisée dans l'invention, l'évolution de la PIE et l'évolution de la microvascularisation sont évaluées 24 heures et 48 heures après le retrait du patch. Le tableau suivant donne les résultats obtenus :

| | PIE | | Velocimétrie laser Doppler | |
|---|---|---|---|---|
| | 24 h | 48 h | 24 h | 48 h |
| Peau nue | 53,4 | 53 | 206 | 72 |
| Composition selon l'invention | 51 | 42,2 | 131 | 36 |
| % d'évolution | - 5 % | - 20 % | - 36 % | - 50 % |

Ces résultats montrent que la composition utilisée dans l'invention a un effet curatif sur l'inflammation.

## Revendications

1. Utilisation d'une dispersion aqueuse de globules huileux pourvus chacun d'un enrobage cristal liquide monolamellaire ou oligolamellaire, comme actif ayant des propriétés anti-inflammatoires, pour la préparation d'une composition dermatologique destinée à prévenir et/ou traiter une irritation cutanée et/ou à traiter les peaux sensibles et/ou à prévenir et/ou combattre les désordres cutanés faisant intervenir un processus inflammatoire et/ou à prévenir et/ou traiter un érythème solaire, le dit enrobage cristal liquide étant obtenu :
1) soit à partir d'au moins un agent tensioactif lipophile de HLB 2 à 5, d'au moins un agent tensioactif hydrophile de HLB 8 à 12 et d'au moins un acide gras, la dispersion contenant un agent basique à l'état dissous dans une phase aqueuse ;
2) soit à partir d'au moins un agent tensioactif lipophile de HLB 2 à 5, d'au moins un agent tensioactif hydrophile de HLB 8 à 12 et d'au moins un lipide amphiphile ionique conférant à la composition un pH allant de 5,5 à 7,5.

2. Utilisation selon la revendication 1, caractérisée par le fait que l'acide gras comporte au moins une chaîne grasse saturée ayant plus de 12 atomes de carbone, de préférence de 16 à 22 atomes de carbone.

3. Utilisation selon la revendication précédente, caractérisée par le fait que l'acide gras est l'acide stéarique.

4. Utilisation selon l'une quelconque des revendications précédentes, caractérisée par le fait que l'agent basique est choisi dans le groupe comprenant la soude, la triéthanolamine, la lysine et l'arginine.

5. Utilisation selon l'une quelconque des revendications précédentes, caractérisée par le fait que le lipide amphiphile ionique est choisi dans le groupe comprenant les lipides anioniques neutralisés, les lipides amphotères et les dérivés alkylsulfoniques.

6. Utilisation selon la revendication précédente, caractérisée par le fait que le lipide amphiphile ionique est choisi parmi les sels mono- et disodiques de l'acide N-stéaroyl glutamique.

7. Utilisation selon l'une quelconque des revendications précédentes, caractérisée par le fait que l'agent tensioactif lipophile et l'agent tensioactif hydrophile comportent chacun au moins une chaîne grasse saturée ayant plus de 12 atomes de carbone, de préférence de 16 à 22 atomes de carbone.

8. Utilisation selon l'une quelconque des revendications précédentes, caractérisée par le fait que l'agent tensioactif lipophile est le distéarate de saccharose.

9. Utilisation selon l'une quelconque des revendications précédentes, caractérisée par le fait que l'agent tensioactif hydrophile est le monostéarate de sorbitane polyoxyéthyléné à 4 moles d'oxyde d'éthylène.

10. Utilisation selon l'une quelconque des revendications précédentes, caractérisée par le fait que les globules huileux enrobés représentent de 5 à 50 % et de préférence de 10 à 40% en poids par rapport au poids total de la composition.

11. Utilisation selon l'une quelconque des revendications précédentes, caractérisée par le fait que la composition contient en outre une substance choisie parmi les agents antioxydants, antiradicaux libres, mélanorégulateurs, accélérateurs de bronzage, dépigmentants, de coloration de la peau, liporégulateurs, amincissants, anti-acnéiques, antiséborrhéiques, anti-vieillissement, antirides, anti-UV, kératolytiques, anti-inflammatoires, rafraîchissants, cicatrisants, protecteurs vasculaires, antibactériens, antifongiques, antitranspirants, déodorants, conditionneurs des cheveux, immunomodulateurs, nourrissants, les huiles essentielles et les parfums.

12. Utilisation selon l'une quelconque des revendications précédentes, caractérisée par le fait que la composition contient en outre un ou plusieurs composés hydrophiles ou lipophiles cosmétiquement ou dermatologiquement actifs, libres ou encapsulés dans des vésicules.

## Patentansprüche

1. Verwendung einer wäßrigen Dispersion von Ölkügelchen, die mit einer monolamellaren oder oligolamellaren flüssigkristallinen Hülle versehen sind, als Wirkstoff mit entzündungshemmenden Eigenschaften für die Herstellung einer dermatologischen Zusammensetzung, die für die Vorbeugung vor und/oder die Behandlung von Hautreizungen und/oder die Behandlung von empfindlicher Haut und/oder die Vorbeugung vor und/oder die Bekämpfung von Hautkrankheiten, an denen ein entzündlicher Prozeß beteiligt ist, und/oder für die Vorbeugung vor und/oder die Behandlung des Erythema solare vorgesehen ist, wobei die flüssigkristalline Hülle erhältlich ist
1) aus mindestens einem lipophilen grenzflächenaktiven Stoff mit einem HLB-Wert von 2 bis 5, mindestens einem hydrophilen grenzflächenaktiven Stoff mit einem HLB-Wert von 8 bis 12 und mindestens einer Fettsäure, wobei die Dispersion ein basisches Mittel im gelösten Zustand in einer wäßrigen Phase enthält, oder
2) aus mindestens einem lipophilen grenzflächenaktiven Stoff mit einem HLB-Wert von 2 bis 5, mindestens einem hydrophilen grenzflächenaktiven Stoff mit einem HLB-Wert von 8 bis 12 und mindestens einem ionischen amphiphilen Lipid, das dafür sorgt, daß die Zusammensetzung einen pH-Wert von 5,5 bis 7,5 aufweist.

2. Verwendung nach Anspruch 1, dadurch gekennzeichnet, daß die Fettsäure mindestens eine gesättigte Fettkette mit mehr als 12 Kohlenstoffatomen, vorzugsweise 16 bis 22 Kohlenstoffatomen, aufweist.

3. Verwendung nach dem vorhergehenden Anspruch, dadurch gekennzeichnet, daß es sich bei der Fettsäure um Stearinsäure handelt.

4. Verwendung nach einem der vorhergehenden Ansprüche, dadurch gekennzeichnet, daß das basische Mittel unter Natriumhydroxid, Triethanolamin, Lysin und Arginin ausgewählt wird.

5. Verwendung nach einem der vorhergehenden Ansprüche, dadurch gekennzeichnet, daß das ionische amphiphile Lipid unter neutralisierten anionischen Lipiden, amphoteren Lipiden und Alkylsulfon-Derivaten ausgewählt wird.

6. Verwendung nach dem vorhergehenden Anspruch, dadurch gekennzeichnet, daß das ionische amphiphile Lipid unter dem Mono- und dem Dinatriumsalz der N-Stearoylglutaminsäure ausgewählt wird.

7. Verwendung nach einem der vorhergehenden Ansprüche, dadurch gekennzeichnet, daß der lipophile grenzflächenaktive Stoff und der hydrophile grenzflächenaktive Stoff jeweils mindestens eine gesättigte Fettkette mit mehr als 12 Kohlenstoffatomen, vorzugsweise 16 bis 22 Kohlenstoffatomen, aufweisen.

8. Verwendung nach einem der vorhergehenden Ansprüche, dadurch gekennzeichnet, daß es sich bei dem lipophilen grenzflächenaktiven Stoff um Saccharosedistearat handelt.

9. Verwendung nach einem der vorhergehenden Ansprüche, dadurch gekennzeichnet, daß es sich bei dem hydrophilen grenzflächenaktiven Stoff um Sorbitanmonostearat handelt, das mit 4 mol Ethylenoxid polyethoxyliert ist.

10. Verwendung nach einem der vorhergehenden Ansprüche, dadurch gekennzeichnet, daß der Anteil der umhüllten Ölkügelchen 5 bis 50 Gew.-% und vorzugsweise 10 bis 40 Gew.-%, bezogen auf das Gesamtgewicht der Zusammensetzung, beträgt.

11. Verwendung nach einem der vorhergehenden Ansprüche, dadurch gekennzeichnet, daß die Zusammensetzung außerdem eine Substanz enthält, die unter Antioxidantien, Mitteln gegen freie Radikale, Melanoregulatoren, Bräunungsbeschleunigern, Depigmentierungsmitteln, Hautfärbemitteln, fettregulierenden Mitteln, schlankermachenden Mitteln, Antiaknemitteln, Antiseborrhöika, Mitteln gegen die Alterung, Mitteln gegen Falten, UV-Schutzmitteln, Keratolytika, entzündungshemmenden Mitteln, Erfrischungsmitteln, Wundheilungsmitteln, Mitteln für den Gefäßschutz, antibakteriellen Mitteln, Antipilzmitteln, Antitranspirantien, Deodorants, Haarkonditioniermitteln, immunmodulierenden Mitteln, Nährstoffen, essentiellen Ölen und Parfüms ausgewählt wird.

12. Verwendung nach einem der vorhergehenden Ansprüche, dadurch gekennzeichnet, daß die Zusammensetzung außerdem eine oder mehrere kosmetisch oder pharmazeutisch akzeptable, freie oder in Vesikeln verkapselte hydrophile oder lipophile Verbindungen enthält.

## Claims

1. Use of an aqueous dispersion of oily globules, each provided with a monolamellar or oligolamellar liquid crystal coating, as an active agent with anti-inflammatory properties, for the preparation of a dermatological composition intended for preventing and/or treating a skin irritation and/or for treating sensitive skin and/or for preventing and/or for combating skin disorders involving an inflammatory process and/or for preventing and/or treating a solar erythema, the said liquid crystal coating being obtained:
1) either from at least one lipophilic surfactant with an HLB value of from 2 to 5, at least one hydrophilic surfactant with an HLB value of from 8 to 12 and at least one fatty acid, the dispersion containing a basic agent dissolved in an aqueous phase;
2) or from at least one lipophilic surfactant with an HLB value of from 2 to 5, at least one hydrophilic surfactant with an HLB value of from 8 to 12 and at least one ionic amphiphilic lipid giving the composition a pH ranging from 5.5 to 7.5.

2. Use according to Claim 1, characterized in that the fatty acid contains at least one saturated fatty chain having more than 12 carbon atoms, preferably from 16 to 22 carbon atoms.

3. Use according to the preceding claim, characterized in that the fatty acid is stearic acid.

4. Use according to any one of the preceding claims, characterized in that the basic agent is chosen from the group comprising sodium hydroxide, triethanolamine, lysine and arginine.

5. Use according to any one of the preceding claims, characterized in that the ionic amphiphilic lipid is chosen from the group comprising neutralized anionic lipids, amphoteric lipids and alkylsulphonic derivatives.

6. Use according to the preceding claim, characterized in that the ionic amphiphilic lipid is chosen from the mono- and disodium salts of N-stearoylglutamic acid.

7. Use according to any one of the preceding claims, characterized in that the lipophilic surfactant and the hydrophilic surfactant each contain at least one saturated fatty chain having more than 12 carbon atoms, preferably from 16 to 22 carbon atoms.

8. Use according to any one of the preceding claims, characterized in that the lipophilic surfactant is sucrose distearate.

9. Use according to any one of the preceding claims, characterized in that the hydrophilic surfactant is sorbitan monostearate polyoxyethylenated with 4 mol of ethylene oxide.

10. Use according to any one of the preceding claims, characterized in that the coated oily globules represent from 5 to 50%, and preferably from 10 to 40%, by weight relative to the total weight of the composition.

11. Use according to any one of the preceding claims, characterized in that the composition also contains a substance chosen from antioxidants, anti-free-radical agents, melanoregulators, tanning accelerators, depigmenting agents, skin-colouring agents, liporegulators, slimming agents, anti-acne agents, antiseborrhoeic agents, anti-ageing agents, anti-wrinkle agents, anti-UV agents, keratolytic agents, anti-inflammatory agents, refreshing agents, cicatrizing agents, vascular protection agents, antibacterial agents, antifungal agents, antiperspirants, deodorants, hair conditioners, immunomodulators, nourishing agents, essential oils and fragrances.

12. Use according to any one of the preceding claims, characterized in that the composition also contains one or more cosmetically or dermatologically active, hydrophilic or lipophilic compounds, which are free or encapsulated in vesicles.
